# EUROPÄISCHE PATENTSCHRIFT

(11) **EP 2 435 034 B1**
(45) Veröffentlichungstag und Bekanntmachung des Hinweises auf die Patenterteilung: **23.10.2013**
(21) Anmeldenummer: 10726409.5
(22) Anmeldetag: 31.05.2010
(51) Int. Cl.: A61K 31/122, A61P 31/06, A61P 31/00, A61P 31/16, C07C 49/755, C07C 50/34

(54) **VERWENDUNG VON ANTHRACEN-DERIVATEN ALS ANTIINFEKTIVA**
USE OF ANTHRACENE DERIVATIVES AS ANTI-INFECTIVES
UTILISATION DE DÉRIVÉS DE L'ANTHRACÈNE EN TANT QU'ANTI-INFECTIEUX

(30) Priorität: 29.05.2009 AT 8432009
(43) Veröffentlichungstag der Anmeldung: 04.04.2012
(73) Patentinhaber: Sealife Pharma GmbH, 3430 Tulln (AT)
(72) Erfinder: PRETSCH, Alexander, A-2002 Großmugl (AT); PROKSCH, Peter, 52428 Jülich (DE); DEBBAB, Abdessamad, 40215 Düsseldorf (DE)
(74) Vertreter: Ellmeyer, Wolfgang
(86) Internationale Anmeldenummer: PCT/AT2010/000189
(87) Internationale Veröffentlichungsnummer: WO 2010/135758

(56) Entgegenhaltungen:
- DEBBAB ABDESSAMAD ET AL: "Bioactive metabolites from the endophytic fungus Stemphylium globuliferum isolated from Mentha pulegium." JOURNAL OF NATURAL PRODUCTS APR 2009 LNKD- PUBMED:19271717, Bd. 72, Nr. 4, April 2009 (2009-04), Seiten 626-631, XP002596582 ISSN: 1520-6025
- OKAMURA NOBUYUKI ET AL: "Altersolanol-related compounds from the culture liquid of Alternaria solani" PHYTOCHEMISTRY (OXFORD), Bd. 42, Nr. 1, 1996, Seiten 77-80, XP002596583 ISSN: 0031-9422

## Beschreibung

Die vorliegende Erfindung betrifft Anthracen-Derivate zur Verwendung als Antiinfektiva, insbesondere gegen mehrfach wirkstoffresistente Erreger, sowie ein Verfahren zu ihrer Herstellung.

### STAND DER TECHNIK

Durch den höchst erfolgreichem Einsatz antimikrobieller Arzneimittel wurde in den späten 1960er- und frühen 1970er-Jahren angenommen, dass Infektionskrankheiten fortan keine Gefahr mehr darstellen würden. Dies sollte sich als schwerer Irrtum herausstellen, zumal die Mikroben 40 Jahre danach eine größere Bedrohung als jemals zuvor darstellen, weswegen ein dringender Bedarf an neuen antimikrobiellen Wirkstoffen besteht. Heutzutage sind Infektionskrankheiten die dritthäufigste Todesursache in den USA und die zweithäufigste weltweit. Für die meisten dieser Fälle sind unwirksame antimikrobielle Arzneimittel verantwortlich, und die Resistenz mancher Bakterien und Pilze gegen diese Wirkstoffe stellt in unserer Gesellschaft ein ernstes Problem dar. Statistischen Daten aus den USA zufolge wird der Großteil der im Krankenhaus erlittenen Infektionen (den sog. nosokomialen Infektionen) von wenigen Bakterienspezies hervorgerufen, nämlich von *Enterococcus faecium, Staphylococcus aureus, Klebsiella pneumoniae, Acinetobacter baumanii, Pseudomonas aeruginosa* und *Enterobacter sp.,* die nach ihren Anfangsbuchstaben als "ESKAPE"-Pathogene bezeichnet werden (Boucher et al., "IDSA Report on Development Pipeline", CID 2009:48, Infectious Disease Society of America, 1. Januar 2009). Mit diesem Begriff soll freilich gleichzeitig ausgedrückt werden, dass sich diese resistenten Erreger der Wirkung antibakterieller Arzneimittel entziehen (engl.: *to escape*), zumal Antibiotikaresistenz auf molekularer Ebene nichts anderes bedeutet als, dass ein Mikroorganismus die Fähigkeit erlangt hat, sich der wachstumshemmenden oder bakteriziden Wirkung einer antimikrobiellen Substanz zu widersetzen. Das heißt, die Substanz wird klinisch unwirksam. So bedeutet etwa "methicillinresistenter *Staphylococcus aureus"* (auch als MRSA abgekürzt, wiewohl dieselbe Abkürzung auch in allgemeinerer Weise für "multiresistenten *Staphylococcus aureus"* gebraucht wird), dass die Verwendung von β-lactamen in der Therapie von *S*. *aureus* unwirksam ist, während beispielsweise Glykopeptide zumeist Wirkung zeigen. Es ist daher unbedingt erforderlich die Wirkung von neuen Antünfektiva gegen multiresistente Stämmen zu testen, da Wirksamkeit gegen dafür empfindliche Spezies oder Stämme nicht zwangsläufig auch Wirksamkeit gegen multiresistente Bakterien bedeutet, wie auch beispielsweise ein gegen grampositive Bakterien wirksames Mittel nicht notwendigerweise gegen gramnegative Bakterien wirkt oder umgekehrt (siehe u.a. Boucher et al., s.o.).

Neben Bakterien und Pilzen fehlen gegenwärtig aber auch wirksame Strategien gegen respiratorische Viren. Zumeist werden lediglich die Symptome geheilt, ohne das Virus selbst zu bekämpfen. Eine Lösung für die Zukunft könnten vor allem Wirkstoffkombinationen sein.

In der Vergangenheit wurden in endophytischen Pilzen zahlreiche Substanzen zur Bekämpfung der Vermehrung von Mikroorganismen gefunden. Diese Substanzen zeigen in einer Reihe von Fällen sehr gute antibakterielle, antifungale und antivirale Aktivität und könnten für zahlreiche Anwendungen eingesetzt werden (G. A. Strobel, Crit. Rev. Biotechnol. 22, 315-333 (2002)). Aufgrund der Tatsache, dass diesbezügliche Forschung überwiegend akademisch war und nicht direkt die Entwicklung neuer Wirkstoffe zum Ziel hatte, sind heute kaum entsprechende Arzneimittel auf dem Markt. Insbesondere das Screenen gegen resistente Mikroben wurde in der Vergangenheit vernachlässigt, so dass nur einige wenige Substanzen gegen arzneimittelresistente Mikroben gestestet wurden. Noch schlimmer ist die Lage bei respiratorischen Viren, gegen die bisher nahezu keine Substanzen gescreent wurden.

Bereits im Jahre 1969 beschrieb A. Stoessl, Can. J. Chem. 47, 767 (1969), die Isolierung eines neuen metabolischen Pigments aus *Alternaria solani,* einem Schimmelpilz, der die sog. Dürrfleckenkrankheit bei Kartoffeln hervorruft. Dieses Pigment wurde Altersolanol A genannt und seine Struktur wie folgt indentifiziert:

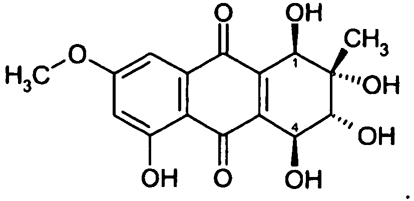

Der chemische Name dieses Anthrachinons lautet demnach 7-Methoxy-2-methyl-(1*R*,2*S*,3*R*,4*S*)-1,2,3,4-tetrahydro-1,2,3,4,5-pentahydroxyanthracen-9,10-dion.

In weiterer Folge wurde dieses Pigment sowie mehrere Isomere und Derivate davon auch in weiteren *Alternaria*-Spezies (z.B. *Alternaria porri*) und in manchen anderen Pilzgattungen nachgewiesen (siehe beispielsweise R. Suemitsu et al., Agric. Biol. Chem. 45(10), 2363-2364 (1981)). Die Mehrzahl der Isomere und Derivate entspricht den folgenden allgemeinen Formeln (1) oder (2): 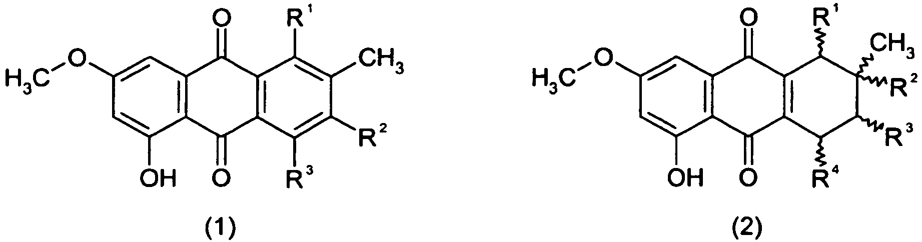 wobei R¹ bis R⁴ jeweils H oder OH sein können, was im Falle von OH in Formel (2) jeweils ein chirales Kohlenstoffatom ergibt, das sowohl in *R*- als auch in *S*-Konfiguration vorliegen kann.

In weiterer Folge wurden auch Dimere, d.h. Bisanthrachinone, als Metaboliten entdeckt, und das erstmals in *Alternaria porri,* einem weiteren Vertreter der Gattung der *Alternariae,* der u.a. die Purpurfleckenkrankheit bei Zwiebelgemüse hervorruft, weswegen diese Dimere Alterporriole genannt wurden. Sie entsprechen beispielsweise der folgenden Formel (3): 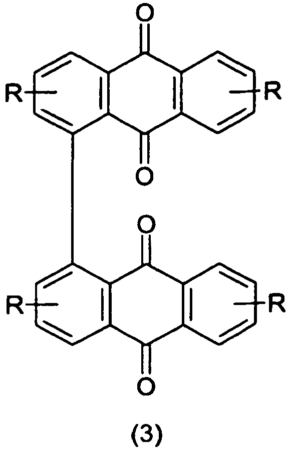 wobei ähnlich vielfältige Substitutions- und Hydrierungsmuster an den aromatischen Ringen möglich sind wie für die "monomeren" Altersolanole. Aufgrund eingeschränkter Freiheit der Rotation um die Achse der chemischen Bindung zwischen den Anthrachinon-Kernen liegen zahlreiche Alterporriol-Derivate in Form zweier Atropisomere vor.

Für manche als Altersolanole bzw. Alterporriole bezeichnete Verbindungen existieren Berichte über ihre Wirksamkeit gegen bestimmte Mikroorganismen, während andere als nicht antiinfektiv beschrieben wurden. So wurden etwa von Aly et al., Phytochemistry 69, 1716-1725 (2009), bioaktive Metaboliten endophytischer Pilze der Gattung *Ampelomyces* sowie deren antiinfektive Wirkung untersucht. Dabei wurde unter anderem festgestellt, dass Altersolanol J, die Alterporriole D und E (Atropisomere), sowie die mit Altersolanolen eng verwandte Verbindung Ampelanol keine Aktivität gegen Bakterien und Pilze zeigen, während Altersolanol A die wirksamste der getesteten Substanzen war. Weiters offenbaren beispielsweise Okamura et al., Phytochemistry 42(1), 77-80 (1996), keinerlei Wirkung von Tetrahydroaltersolanol B gegen grampositive Bakterien und die gramnegative Spezies *Pseudomonas aeruginosa,* während sich Altersolanol A und G gegen *Micrococcus luteus, Staphylococcus aureus* und *Pseudomonas aeruginosa* als wirksam erwiesen. Yagi et al., Phytochemistry 33(1), 87-91 (1993), berichten über die antimikrobielle Aktivität der Altersolanole A, B, C und E, während die Altersolanole D und F in demselben Test völlig unwirksam waren. Weiters offenbaren Okamura et al. in Phytochemistry 34(4), 1005-1009 (1993), Folgendes: Wirksamkeit von Altersolanol A und Altersolanol G gegen grampositive Bakterien und *Pseudomonas aeruginosa,* sehr schwache Wirksamkeit von Alterporriol A, B und D, keine Wirksamkeit von Altersolanol H sowie keine Wirksamkeit irgendeiner dieser Verbindungen gegen Candida-Hefepilze. In der US-Pa-tentanmeldung Nr. 2007/258913 werden die atropisomeren Altersolanole D und E hingegen, wenn auch nur ganz allgemein, als geeignete Verbindungen zur Verhinderung der Bildung eines Biofilms in der Mundhöhle offenbart, ohne allerdings konkrete Daten bezüglich ihrer Wirksamkeit anzugeben, und Debbab et al., J. Nat. Prod. 72, 626-631 (2009), beschreiben eine gewisse zytotxische Wirksamkeit der Alterporriole G+H gegen Mäuse-Lymphomzellen.

Somit ist es jedenfalls unmöglich vorherzusagen, ob ein bestimmtes Altersolanol- bzw. Alterporriol-Isomer oder -Derivat antiinfektive Wirkung zeigt oder nicht, geschweige denn gegen welche Gattungen oder gar Spezies von Mikroorganismen.

Das Ziel der Erfindung war vor diesem Hintergrund die Identifikation, Isolierung und Herstellung neuer Substanzen zur Verwendung als antiinfektive Wirkstoffe in pharmazeutischen Zusammensetzungen, vor allem von Verbindungen, die Aktivität gegen mehrfach wirkstoffresistente ("multiple drug resistant", MDR-) Erreger zeigen.

Die Erfinder konnten nun im Verlauf ihrer Forschungen mehrere Substanzen - zum Teil mit bisher unveröffentlichter Struktur, d.h. neue chemische Verbindungen - identifizieren, die gute Aktivität gegen Mikroorganismen, aber auch gegen respiratorische Viren, insbesondere auch gegen MDR-Erreger, zeigen. Während in der parallelen, gleichzeitig eingereichten österreichischen Patentanmeldung mit der Anmeldenummer A 842/09 zwei neue Verbindungen, nämlich jeweils ein Altersolanol- und ein Alterporriol-Derivat bereitgestellt werden, bezieht sich die vorliegende Erfindung auf die Verwendung mehrerer Verbindungen, deren Struktur zwar bereits aus der Literatur bekannt ist, die aber eine bislang unbekannte Wirkung zeigen.

### OFFENBARUNG DER ERFINDUNG

Konkret haben die Erfinder herausgefunden, dass die nachstehenden Anthracen-Derivate mitunter ausgezeichnete Aktivität gegen verschiedene Bakterien, Pilze und Viren zeigen, weswegen die vorliegende Erfindung in einem ersten Aspekt diese Derivate zur Verwendung als Antiinfektiva betrifft:
a) (2*R*,3*S*,10*R*)-2,3,5,10-Tetrahydroxy-6-methoxy-3-methyl-1,2,3,4,4a,9a-hexahydro-9H,10H-anthracen-9-on (Tetrahydroaltersolanol B) der Formel (4) 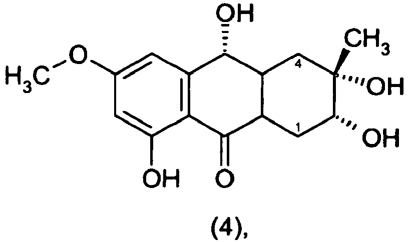
b) (1*R*,2*R*,3*R*,4a*S*,9a*R*,10*R*)-1,2,3,5,10-Pentahydroxy-7-methoxy-2-methyl-1,2,3,4, 4a,9a-hexahydro-9H,10H-anthracen-9-on (Altersolanol K) der Formel (5) 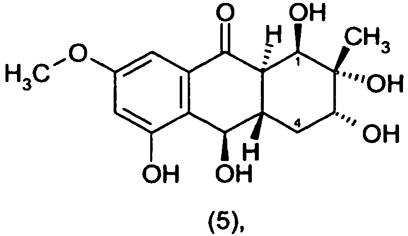
c) (2*R*,3*R*,4*R*,4a*S*,9a*S*,10*R*)-2,3,4,8,10-Pentahydroxy-6-methoxy-3-methyl-1,2,3,4, 4a,9a-hexahydro-9H,10H-anthracen-9-on (Altersolanol L) der Formel (6) 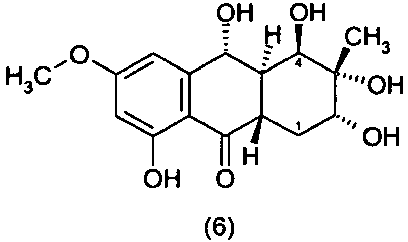 und
d) 8-(1,7-Dihydroxy-6-methyl-3-methoxy-9,10-dioxo-9H,10H-anthracen-2-yl)-(1*S*,2*S*,3*R*,4*S*)-1,2,3,4,5-pentahydroxy-7-methoxy-2-methyl-1,2,3,4-tetrahydro-9H,10H-anthracen-9,10-dion (Atropisomere Alterporriol G und H) der Formel (7)

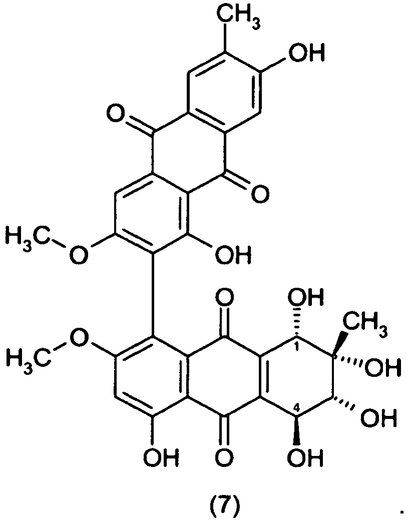

Vorzugsweise dienen diese Anthracen-Derivate zur Verwendung als Antiinfektiva gegen mehrfach wirkstoffresistente ("multiple drug resistant", MDR-) Erreger, da sie gerade hier sehr gute bis ausgezeichnete Wirksamkeit zeigen. Die Strukturen dieser fünf Substanzen wurden in der Vergangenheit zwar bereits veröffentlicht, über ihre Wirkung ist jedoch praktisch nichts bekannt.

Für Verbindung (4), Tetrahydroaltersolanol B, beschreiben Okamura et al. (s.o.), wie zuvor erwähnt, sogar Unwirksamkeit gegen grampositive Bakterien und die gramnegative Spezies *Pseudomonas aeruginosa.* Zu den Verbindungen (5), (6) und (7), d.h. Altersolanol K, Altersolanol L und den Atropisomeren Alterporriol G und H, wurden hingegen noch keinerlei Aktivitätstests offenbart. Alle wurden von Miterfindem des vorliegenden Anmeldungsgegenstands erstmalig aus *Stemphylium globuliferum* isoliert, wie dies auch auf der Website des Journal of Natural Products am 9. März 2009 publiziert wurde (Debbab et al., s.o.).

Die Erfindung betrifft weiters (2*R*,3*S*,4a*S*,9a*S*,10*R*)-2,3,5,8,10-Pentahydroxy-6-methoxy-3-methyl-1,2,3,4,4a,9a-hexahydro-9H,10H-anthracen-9-on (8-Hydroxytetrahydro-altersolanol B) der Formel (8) 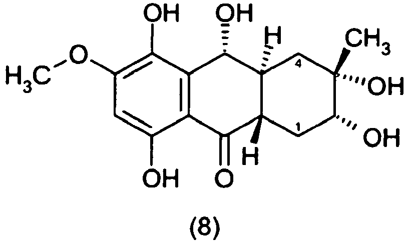 zur Verwendung als Antiinfektivum gegen MDR-Erreger. Auch die Struktur dieser Verbindung ist bereits bekannt. Bezüglich ihrer Aktivität wurde jedoch bisher ausschließlich von Xu et al., J. Antibiot. 61(7), 415-419 (2008), und von Sommart et al., Chemical & Pharmaceutical Bulletin 56(12), 1687-1690 (2008), geringe Wirksamkeit gegen *Staphylococcus aureus* bzw. methicillinresistenten *Staphylococcus aureus* offenbart. Die Erfinder haben hingegen herausgefunden, dass Verbindung (8) tatsächlich sehr gute Aktivität gegen methicillinresistenten *Staphylococcus aureus* und sogar ausgezeichnete Wirksamkeit gegen *Streptococcus pneumoniae* besitzt.

Die obigen Verbindungen der Formeln (4) bis (7) dienen daher vorzugsweise zur Verwendung gegen mehrfach wirkstoffresistente Stämme von methicillinresistentem *Staphylococcus aureus* (MRSA), *Staphylococcus epidermis, Streptococcus pneumoniae, Acinetobacter baumanii, Enterococcus faecalis* bzw. *faecium, E. coli, Klebsiella sp., Pseudomonas aeruginosa, Aspergillus sp.* sowie respiratorische Viren aus der Gruppe der humanen Rhinoviren und der respiratorischen Synzytial-Viren, und die Verbindung der Formel (8) vorzugsweise gegen dieselben Erreger mit Ausnahme von *Staphylococci,* d.h. gegen *Streptococcus pneumoniae, Acinetobacter baumanii, Enterococcus faecatis* bzw. *faecium, E. coli, Klebsiella sp., Pseudomonas aeruginosa, Aspergillus sp.* sowie ebenfalls respiratorische Viren aus der Gruppe der humanen Rhinoviren und der respiratorischen Synzytial-Viren, was durch die späteren Ausführungsbeispiele im Detail belegt wird.

Die vorliegende Erfindung betrifft in einem weiteren Aspekt auch ein Verfahren zur Herstellung der Verbindungen der Formeln (4) bis (8), das darin besteht, einen die Verbindung oder einen Vorläufer davon produzierenden Mikroorganismus unter Wachstumsbedingungen zu fermentieren und die jeweilige Verbindung, gegebenenfalls nach Zerstörung der Zellen des Mikroorganismus zur Erhöhung der Ausbeute, aus der Kultur zu gewinnen. In einem solchen Fermentationsverfahren wird als Mikroorganismus erfindungsgemäß ein reiner Stamm von *Nigrospora sp.* eingesetzt, da die Erfinder mit dieser Spezies die besten Ausbeuten erzielen konnten. Alternativ dazu können jedoch auch beliebige andere Mikroorganismen, die in der Lage sind, die jeweilige Verbindung - oder auch eine Vorstufe davon - zu produzieren, z.B. *Alternaria sp.,* zur Fermentation eingesetzt werden. Werden durch die Fermentation Vorstufen erhalten, können diese nach beliebigen, dem einschlägigen Fachmann auf dem Gebiet der organischen Synthese wohlbekannten Verfahren in die gewünschten Verbindungen der Formeln (4) bis (8) übergeführt werden, wobei gegebenenfalls auch Verfahrensschritte unter Enzymkatalyse eingesetzt werden können, um höhere Enantioselektivität zu gewährleisten.

Beispielsweise kann etwa *Alternaria sp.* kultiviert werden, um aus der Fermentationsbrühe Altersolanol B - oder auch ein anderes Mitglied der Altersolanol-Familie - zu erhalten, das durch Hydrierung der nichtaromatischen Doppelbindung zwischen den Kohlenstoffatomen 4a und 9a sowie der Ketogruppe an Position 9 in die gewünschte Verbindung einer der Formeln (4) bis (6) oder (8), z.B. in Tetrahydroaltersolanol B, oder ein Stereoisomer davon übergeführt werden kann. Ein weiterer Syntheseweg besteht in der Abspaltung einer OH-Gruppe zusammen mit dem benachbarten Wasserstoffatom, d.h. Dehydratisierung, z.B. am hydrierten Ring von Altersolanol A oder B, um eine Doppelbindung zwischen den ensprechenden C-Atomen sowie zwei prochirale Zentren an diesen Positionen zu erhalten. Anschließend kann diese Doppelbindung enzymatisch rehydratisiert oder aber cis- oder trans-dihydroxyliert werden, was bei geeigneter Wahl der Stereospezifität des Enzyms (z.B. Hydratase, Peroxygenase) oder des Oxidationsmittels (z.B. Permanganat) die gewünschte Verbindung ergibt. Die Alterporriole G und H können beispielsweise auch durch chemische (wiederum gegebenenfalls enzymatische) Anbindung des entsprechenden Anthrachinon-Substituenten an Position 8 von Altersolanol M - dem entsprechenden, an Position 3 acetylierten, "monomeren" Anthrachinon - erhalten werden, das gegebenenfalls davor geeignet derivatisiert wurde, wonach die Acetylgruppe von der OH-Gruppe an Position 3 hydrolytisch abgespalten wird.

Geeignete Enzyme für die Syntheseschritte zur Umsetzung von Vorläufern der gewünschten Verbindung können in manchen Fällen ebenfalls aus dem zur Fermentation eingesetzten oder auch aus einem anderen Mikroorganismus isoliert werden. Letzterer Fall kann sinnvoll sein, wenn beispielsweise ein Mikroorganismus zwar das gewünschte Produkt produziert, aber z.B. in so geringen Mengen oder so stark verunreinigt, dass es wirtschaftlicher ist, durch Kultivierung eines anderen Stamms (oder sogar einer anderen Spezies oder Gattung) einen Vorläufer des Produkts zu erhalten und diesen mittels enzymatischer Synthese zur Zielverbindung umzusetzen.

Die Gewinnung der jeweiligen Verbindung (4) bis (8) erfolgt gemäß vorliegender Erfindung jedoch vorzugsweise durch Extraktion und anschließende Isolierung aus einem Rohextrakt einer Kultur, z.B. mittels fraktionierter Kristallisation oder Chromatographieverfahren, noch bevorzugter präparativer HPLC, was bisher erneut die besten Ausbeuten bei gleichzeitig höchster Reinheit ergeben hat. Freilich sind speziell bei Ansätzen in größerem Maßstab auch andere Isolierungsverfahren, wie z.B. direkte fraktionierte Kristallisation des Rohextrakts oder auch Absorptionsmethoden, denkbar. Der Fachmann kann jedoch ohne übermäßiges Experimentieren das für den jeweiligen Kultivierungs- (bzw. Synthese- oder Partialsynthese-) Schritt geeignete Isolierungsverfahren ermitteln.

### BEISPIELE

Die Erfindung wird nachstehend unter Bezugnahme auf konkrete Ausführungsbeispiele näher beschrieben, die jedoch den Schutzumfang nicht einschränken sollen.

Die Verbindungen der Formeln (4) bis (8) wurden durch Kultivierung von *Stemphylium globuliferum* für die Verbindungen (5) bis (7) bzw. *Nigrospora sp.* für die Verbindungen (4) und (8) und anschließende Extraktion erhalten.

### Isolierung der Mikroorganismen

### a) Stemphylium globuliferum

Frische, gesunde Stängel von *Mentha pulegium* (Polei-Minze) wurden zur Isolierung des endophytischen Pilzes verwendet. Die Oberfläche der Stängel wurde mit 70%-igem Ethanol 1 min lang sterilisiert und danach mit sterilem Wasser abgespült, um den Alkohol zu entfernen. Zur Unterscheidung etwaiger verbliebener epiphytischer Pilze von endophytischen Pilzen wurde ein Abdruck der Stängeloberfläche auf Biomalzagar angefertigt. Kleine Gewebeproben aus dem Inneren wurden aseptisch aufgeschnitten und auf Agarplatten gepresst, die zur Unterdrückung von bakteriellem Wachstum ein Antibiotikum enthielten. Die Zusammensetzung des Isoliermediums war folgende: 15 g/l Malzextrakt, 15 g/l Agar und 0,2 g/l Chloramphenicol in destillier-tem Wasser, pH 7,4-7,8). Aus den Kulturen wurde durch wiederholtes Überimpfen auf Malzagarplatten ein reiner Pilzstamm erhalten. Die Pilzkultur wurde nach einer molekularbiologischen Vorschrift mittels DNA-Amplifikation und Sequenzierung der ITS-Region als *Stemphylium globuliferum* identifiziert. Die Sequenzdaten wurden bei GenBank unter der Zugriffsnummer EU859960 hinterlegt.

### b) Nigrospora sp.

Die Isolierung und Identifizierung erfolgte analog zu jener von *Stemphylium globuliferum,* wobei jedoch anstelle der Minzstängel Blätter von *Bruguiera sexangula,* einer Magrovenspezies, verwendet wurden.

### Kultivierung

Zur Kultivierung beider isolierter Mikroorganismen wurden je zwei 1-Liter-Erlenmeyer-Kolben, die jeweils 100 g Reis und 100 ml destilliertes Wasser enthielten, autoklaviert, um ein gequollenes, festes Reismedium zu erhalten. Ein kleiner Teil des obigen Isoliermediums, das den reinen Pilzstamm enthielt, wurde unter sterilen Bedingungen auf das feste Reismedium aufgebracht, und der Pilz wurde bei Raumtemperatur (22 °C) 40 d lang für *Stemphylium globuliferum* bzw. 30 d lang für *Nigrospora* sp. kultiviert.

### Extraktion und Isolierung

Nach 30 bzw. 40 d wurde die Kultur zweimal mit 300 ml Ethylacetat (EtOAc) extrahiert. Der EtOAc-Extrakt wurde bis zur Trockene eingedampft und zwischen n-Hexan und 90%igem wässrigen Methanol (MeOH) verteilt. Die wässrig-methanolische Phase wurde bis zur Trockene eingedampft, und der Rückstand wurde zunächst Säulenchromatographie über eine Sephadex LH-20-Säule mit 100%igem Methanol (MeOH) als Laufmittel unter Detektion mittels Dünnschichtchromatographie (DC) auf Kieselgel F245 (Merck, Darmstadt, Deutschland) unter Verwendung von EtOAc/MeOH/H₂O (77:13:10) als Laufmittel unterzogen wurde. Die die gewünschten Verbindungen enthaltenden Fraktionen wurden vereinigt und semipräparativer HPLC (Merck, Hitachi L-7100) unter Verwendung einer Eurosphere 100-10 C18-Säule (300 x 8 mm, L x i.d.) und eines linearen Wasser-Methanol-Gradienten unterzogen. Auf diese Weise wurden die Verbindungen der Formeln (4) bis (8) in reiner Form erhalten, wobei die Atropisomere Alterporriol G und H nicht getrennt werden konnten.

Die Anteile der einzelnen Verbindungen an der Gesamtausbeute betrugen 49,0 Gew.-% Alterporriol G und H, 10,4 Gew.-% Altersolanol K, 7,8 Gew.% Altersolanol L, 14,6 Gew.-% Tetrahydroaltersolanol B und 18,2 Gew.-% 8-Hydroxytetrahydroaltersolanol B.

Die Identifizierung der Verbindungen erfolgte anhand ihrer ¹H-NMR-, ¹³C-NMR-, HMBC- und COSY-Spektren sowie Vergleich mit den in der erwähnten Literatur zitierten Werten.

### Aktivitätsbestimmung

Die Aktivität der Verbindungen wurden in zwei unterschiedlichen Screeningsystemen getestet. Die antibakterielle und antifungale Aktivität wurde in einem MHK-Test untersucht. MHK steht für die "minimale Hemm-Konzentration" (engl.: MIC für "minimal inhibitory concentration") und bezeichnet die niedrigste Konzentration einer Substanz, bei der mit bloßem Auge keine Vermehrung von Mikroorganismen wahrgenommen werden kann. Bestimmt wird die MHK mit einem so genannten Titerverfahren, bei dem die Substanz ausverdünnt und anschließend der Erreger zugefügt wird.

In der Regel wird so die Konzentration eines Antibiotikums bestimmt, die das Wachstum eines Bakterienstammes gerade noch hemmt. Die MHK wird in Mikrogramm pro Milliliter (µg/ml) angegeben, und die Verdünnungen erfolgen in der Regel in log2-Schritten. Hierin wurde eine Ausgangskonzentration von 250 µg/ml jeweils auf das Doppelte verdünnt, was folglich Testkonzentrationen von 250 µg/ml, 125 µg/ml, 62,5 µg/ml, 31,2 µg/ml, 15,6 µg/ml, 7,8 µg/ml usw. ergab. Niedrigere Werte spiegeln demnach bessere Aktivität als Antiinfektivum wider.

Die Tests wurden nach den vom EUCAST (European Committee for Antimicrobial Susceptibility Testing) geforderten Standards und gemäß den AFST- ("Antifungal Susceptibility Testing") Vorschriften der European Society of Clinical Microbiology and Infectious Diseases (ESCMID) durchgeführt.

Das Screeningsystem für Viren ist ein Infektionssystem, bei dem Wirtszellen *in vitro* infiziert werden und die Testsubstanz vor oder nach der Infektion zugesetzt und ihre Aktivität bestimmt wird. Alle diese Tests wurden gemäß den betriebsintemen Standardvorschriften von SeaLife Pharma zum Arzneimittelscreening durchgeführt, wobei analoge Verdünnungsreihen wie im antibakteriellenlantifungalen Test eingesetzt wurden.

In der nachstehenden Tabelle 1 sind die Testergebnisse bezüglich der antiinfektiven Wirkung gegen einige multiresistente Bakterien und Pilze (die allesamt freundlicherweise von Prof. Georgopulos von der Medizinischen Universität Wien zur Verfügung gestellt worden waren) für Altersolanol M, Alterporriol I und J sowie einige bekannte und strukturell ähnliche Vergleichssubstanzen angeführt. Die Daten sind jeweils Mittelwerte von Mehrfachbestimmungen.

Es ist klar ersichtlich, dass die Verbindungen der Formeln (4) bis (8) gute bis ausgezeichnete Aktivität gegen fünf Bakterienspezies, nämlich *Enterococcus faecalis* bzw. *faecium,* methicillinresistenten *Staphylococcus aureus, Streptococcus pneumoniae, Staphylococcus epidermis* und *Acinetobacter baumanii,* und darüber hinaus auch - wenn auch nur mäßige bis geringe - Aktivität gegen *Aspergillus fumigatus* bzw. *faecalis* zeigen. Als einzige Verbindung in dieser Testreihe zeigte Tetrahydroaltersolanol B auch schwache Wirkung gegen E. coli, *Klebsiella sp.* und *Pseudomonas aeruginosa.*

Als Ergebnis der Aktivitätstest der Verbindungen (4) bis (8) gegen drei verschiedene Spezies respiratorischer Viren, nämlich ein humanes Rhinovirus (hrv), ein respiratorisches Synzytial-Virus (rsv) und Paraflu, die von der ATCC erhalten worden waren, wurde festgestellt, dass beide (bzw. alle drei) Verbindungen bereits ab einer Konzentration von 0,1 µg/ml für 100%igen Schutz der Zellen sorgten.

Somit wurde eindeutig belegt, dass die erfindungsgemäße Verwendung der fünf bekannten Verbindungen mitunter ausgezeichnete Wirksamkeit sowohl gegen mehrfach arzneimittelresistente bakterielle und fungale Erreger als auch gegen respiratorische Viren zeigen und daher breite Anwendung als Antiinfektiva finden können.

**Tabelle 1: MHK-Werte bei multiresistenten Bakterien und Pilzen**

| **Substanzen** | **EC** | **KL** | **PS** | **EK** | **MRSA** | **STR** | **SE** | **AB** | **ASP** |
|---|---|---|---|---|---|---|---|---|---|
| Beispiel 1: Tetrahydroaltersolanol B (4) | 125 | 125 | 125 | | 31,2 | 15,6 | 31,2 | 31,2 | |
| Beispiel 2: Altersolanol K (5) | | | | 31,2 | 31,2 | 31,2 | 31,2 | | 62,5 |
| Beispiel 3: Altersolanol L (6) | | | | 7,8 | 7,8 | 7,8 | 7,8 | 7,8 | 31,2 |
| Beispiel 4: Alterporriol G + H (7) | | | | 7,8 | 7,8 | 7,8 | 7,8 | | 125 |
| Beispiel 5: 8-Hydroxytetrahydroaltersolanol B (8) | | | | 125 | 15,6 | 7,8 | 15,6 | | 31,2 |
| Bostrycin | | | | | 125 | 62,5 | 125 | 125 | |
| Altenusin | 62,5 | | | 62,5 | 31,2 | 31,2 | 31,2 | 62,5 | |
| Alterporriol A + B | | | | 15,6 | 15,6 | 7,8 | 15,6 | 125 | 62,5 |
| Altersolanol A | 62,5 | | | 15,6 | 7,8 | 7,8 | 7,8 | 62,5 | 7,8 |
| Altersolanol J | | 125 | | 31,2 | 62,5 | 15,6 | 62,5 | 125 | 125 |
| Ampelanol | | | | | | | | | |
| Macrosporin | 62,5 | | | | 62,5 | | 62,5 | 125 | |
| Macrosporinsulfat | | | | | | 31,2 | | 125 | |
| Indolcarbaldehyd (Carboxaldehyd) | | | 125 | | | 62,5 | | 125 | |

| | | | | | | | | | |
|---|---|---|---|---|---|---|---|---|---|
| EC *E. coli* KL *Klebsiella sp.* PS *Pseudomonas aeruginosa* EK *Enterococcus faecalis* bzw. *faecium* MRSA methicillinresistenter *Staphylococcus aureus* STR *Streptococcus pneumoniae* SE *Staphylococcus epidermis* AB *Acinetobacter baumanii* ASP *Aspergillus fumigatus* bzw. *faecalis* | | | | | | | | | |

## Patentansprüche

1. Nachstehende Anthracen-Derivate zur Verwendung als Antiinfektiva:
a) (2*R*,3*S*,10*R*)-2,3,5,10-Tetrahydroxy-6-methoxy-3-methyl-1,2,3,4,4a,9a-hexahydro-9H,10H-anthracen-9-on (Tetrahydroaltersolanol B) 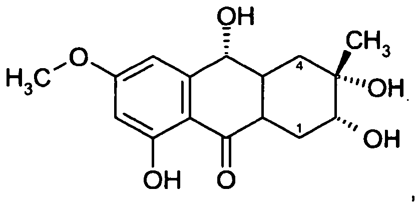
b) (1*R*,2*R*,3*R*,4a*S*,9a*R*,10*R*)-1,2,3,5,10-Pentahydroxy-7-methoxy-2-methyl-1,2,3,4, 4a,9a-hexahydro-9H,10H-anthracen-9-on (Altersolanol K) 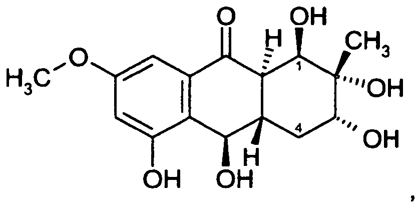
c) (2*R*,3*R*,4*R*,4a*S*,9a*S*,10*R*)-2,3,4,8,10-Pentahydroxy-6-methoxy-3-methyl-1,2,3,4, 4a,9a-hexahydro-9H,10H-anthracen-9-on (Altersolanol L) 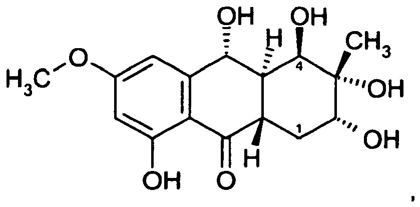
d) 8-(1,7-Dihydroxy-6-methyl-3-methoxy-9,10-dioxo-9H,10H-anthracen-2-yl)-(1*S*,2*S*,3*R*,4*S*)-1,2,3,4,5-pentahydroxy-7-methoxy-2-methyl-1,2,3,4-tetrahydro-9H,10H-anthracen-9,10-dion (Atropisomere Alterporriol G und H)

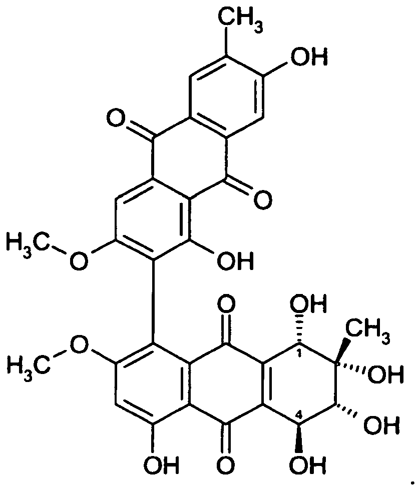

2. Anthracen-Derivat zur Verwendung als Antünfektivum nach Anspruch 1, **dadurch gekennzeichnet, dass** das Anthracen-Derivat zur Verwendung als Antiinfektivum gegen mehrfach wirkstoffresistente ("multiple drug resistant", MDR-) Erreger dient.

3. Anthracen-Derivat zur Verwendung als Antünfektivum nach Anspruch 2, **dadurch gekennzeichnet, dass** das Anthracen-Derivat zur Verwendung als Antiinfektivum gegen mehrfach wirkstoffresistente Stämme von methicillinresistentem *Staphylococcus aureus* (MRSA), *Staphylococcus epidermis, Streptococcus pneumoniae, Acinetobacter baumanii, Enterococcus faecalis* bzw. *faecium, Escherichia coli, Klebsiella sp., Pseudomonas aeruginosa, Aspergillus sp.* sowie respiratorische Viren aus der Gruppe der humanen Rhinoviren und der respiratorischen Synzytial-Viren dient.

4. (2*R*,3*S*,4a*S*,9a*S*,10*R*)-2,3,5,8,10-Pentahydroxy-6-methoxy-3-methyl-1,2,3,4, 4a,9a-hexahydro-9H,10H-anthracen-9-on(8-Hydroxytetrahydroaltersolanol B) 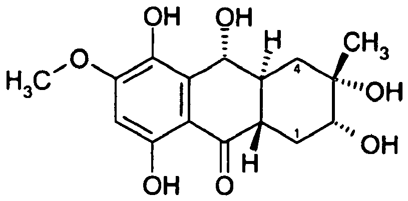 zur Verwendung als Antiinfektivum gegen mehrfach wirkstoffresistente ("multiple drug resistant", MDR-) Erreger, ausgewählt aus mehrfach wirkstoffresistenten Stämmen von *Streptococcus pneumoniae, Acinetobacter baumanii, Enterococcus faecalis* bzw. *faecium, Escherichia coli, Klebsiella sp., Pseudomonas aeruginosa, Aspergillus* sp. sowie respiratorische Viren aus der Gruppe der humanen Rhinoviren und der respiratorischen Synzytial-Viren.

5. Verfahren zur Herstellung einer der in Anspruch 1 oder 4 definierten Verbindungen, **dadurch gekennzeichnet, dass** ein Stamm von *Nigrospora sp.* unter Wachstumsbedingungen fermentiert wird und die Verbindung, gegebenenfalls nach Zerstörung der Zellen des Mikroorganismus, aus der Fermentationsbrühe gewonnen wird.

6. Verfahren nach Anspruch 5, **dadurch gekennzeichnet, dass** die Verbindung durch Extraktion und anschließende Isolierung aus dem Rohextrakt gewonnen wird.

## Claims

1. The following anthracene derivatives for use as anti-infectives:
a) (2*R*,3*S*,10*R*)-2,3,5,10-tetrahydroxy-6-methoxy-3-methyl-1,2,3,4,4a,9a-hexahydro-9H,10H-anthracene-9-one (tetrahydroaltersolanol B) 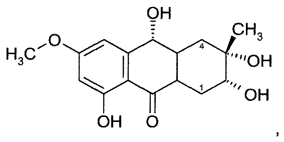
b) (1*R*,2*R*,3*R*,4a*S*,9a*R*,10*R*)-1,2,3,5,10-pentahydroxy-7-methoxy-2-methyl-1,2,3,4, 4a,9a-hexahydro-9H,10H-anthracene-9-one (altersolanol K) 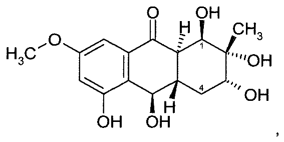
c) (2*R*,3*R*,4*R*,4a*S*,9a*S*,10*R*)-2,3,4,8,10-pentahydroxy-6-methoxy-3-methyl-1,2,3,4, 4a,9a-hexahydro-9H,10H-anthracene-9-one (altersolanol L) 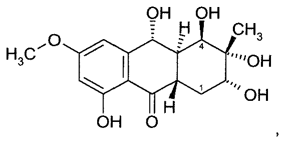
d) 8-(1,7-dihydroxy-6-methyl-3-methoxy-9,10-dioxo-9H,10H-anthracene-2-yl)-(1*S*,2*S*,3*R*,4*S*)-1,2,3,4,5-pentahydroxy-7-methoxy-2-methyl-1,2,3,4-tetrahydro-9H,10H-anthracene-9,10-dione (atropisomers alterporriol G and H)

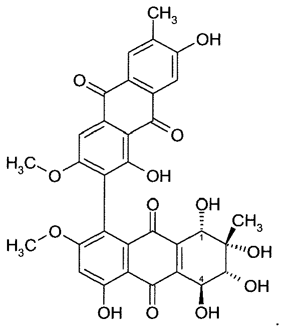

2. The anthracene derivative for use as an anti-infective according to Claim 1 **characterized in that** the anthracene derivative serves for use as an anti-infective against multiple drug-resistant (MDR) pathogens.

3. The anthracene derivative for use as an anti-infective according to Claim 2, **characterized in that** the anthracene derivative serves for use as an anti-infective against multiple drug-resistant strains of methicillin-resistant *Staphylococcus aureus* (MRSA), *Staphylococcus epidermis, Streptococcus pneumoniae, Acinetobacter baumanii, Enterococcus faecalis* or *faecium, Escherichia coli, Klebsiella sp., Pseudomonas aeruginosa, Aspergillus sp.* as well as respiratory viruses from the group of human rhinoviruses and respiratory syncytial viruses.

4. (2*R*,3*S*,4a*S*,9a*S*,10*R*)-2,3,5,8,10-Pentahydroxy-6-methoxy-3-methyl-1,2,3,4,4a,9a-hexahydro-9H,10H-anthracene-9-one (8-hydroxytetrahydroaltersolanol B) 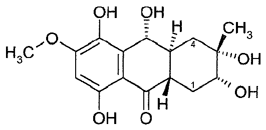 for use as an anti-infective against multiple drug-resistant (MDR) pathogens, selected from multiple-drug resistant strains of *Streptococcus pneumoniae, Acinetobacter baumanii, Enterococcus faecalis* or *faecium, Escherichia coli, Klebsiella sp., Pseudomonas aeruginosa, Aspergillus sp.* as well as respiratory viruses from the group of human rhinoviruses and respiratory syncytial viruses.

5. A method for manufacturing a compound defined in any one of the Claims 1 or 4, **characterized in that** a strain of *Nigrospora sp.* is fermented under growth conditions and the compound is obtained from the fermentation broth, optionally after disruption of the cells of the microorganism.

6. The method according to Claim 5, **characterized in that** the compound is obtained from the crude extract by extraction and subsequent isolation.

## Revendications

1. Les suivants dérivés d'anthracène pour utilisation comme anti-infectieux:
a) (2*R*,3*S*,10*R*)-2,3,5,10-tétrahydroxy-6-méthoxy-3-méthyl-1,2,3,4,4a,9a-hexahydro-9H,10H-anthracène-9-on (tétrahydroaltersolanol B) 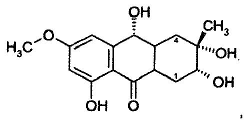
b) (1*R*,2*R*,3*R*,4a*S*,9a*R*,10*R*)-1,2,3,5,10-pentahydroxy-7-méthoxy-2-méthyl-1,2,3,4,4a,9a-hexahydro-9H,10H-anthracène-9-on (altersolanol K) 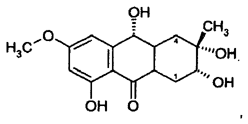
c) (2*R*,3*R*,4*R*,4a*S*,9a*S*,10R)-2,3,4,8,10-pentahydroxy-6-methoxy-3-méthyl-1,2,3,4,4a,9a,hexahydro-9H,10H-anthracène-9-on (altersolanol L) 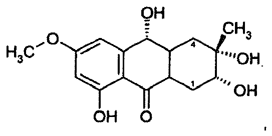
d) 8-(1,7-dihydroxy-6-méthyl-3-méthoxy-9,10-dioxo-9H,10H-anthracène-2-yl)-(1S,2S,3R,4S)-1,2,3,4,5-pentahydroxy-7-méthoxy-2-méthyl-1,2,3,4-tétrahydro-9H,10H-anthracène-9,10-dion (atropisomères alterporriol G et H)

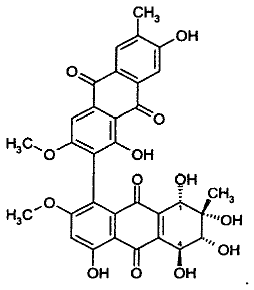

2. Dérivé d'anthracène pour utilisation comme anti-infectieux selon la revendication 1, **caractérisé en ce que** le dérivé d'anthracène est pour utilisation comme anti-infectieux contre des agents pathogènes à résistance polymédicamenteuse («multiple drug resistant», MDR).

3. Dérivé d'anthracène pour utilisation comme anti-infectieux selon la revendication 2, **caractérisé en ce que** le dérivé d'anthracène est pour utilisation comme anti-infectieux contre des souches à résistance polymédicamenteuse de *Staphylococcus aureus* (MRSA), *Staphylococcus epidermis, Streptococcus pneumoniae, Acinetobacter baumanii, Enterococcus faecalis* ou *faecium, Escherichia coli, Klebsiella sp., Pseudomonas aeruginosa, Aspergillus sp.* résistant à la méthicilline ainsi que de virus respiratoires appartenant au groupe des rhinovirus humains et des virus synctiaux.

4. (2*R*, 3*S*,4a*S*,9a*S*,10*R*)-2,3,5,8,10-pentahydroxy-6-méthoxy-3-méthyl-1,2,3,4,4a,9a,hexahydro-9h,10H-anthracène-9-on(8-hydroxytétrahydroaltersolanol B) 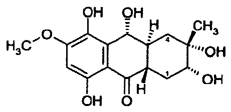 pour utilisation comme anti-infectieux contre des agents pathogènes à résistance polymédicamenteuse (« multiple drug resistant », MDR), sélectionnés parmi des souches à résistance polymédicamenteuse de *Acinetobacter baumanii, Enterococcus faecalis* ou *faecium, Escherichia coli, Klebsiella sp., Pseudomonas aerugino*sa, *Aspergillus sp.* ainsi que de virus respiratoires appartenant au groupe des rhinovirus humains et des virus synctiaux.

5. Procédé pour la production d'un des composés définis dans la revendication 1 ou 4, **caractérisé en ce qu'**une souche de *Nigrospora sp.* est fermentée sous des conditions de croissance et le composé est obtenu à partir du bouillon de fermentation, éventuellement après destruction des cellules du microorganisme.

6. Procédé selon la revendication 5, **caractérisé en ce que** le composé et obtenu par extraction et successivement par isolation à partir de l'extrait brut.
